(19) Europäisches Patentamt
European Patent Office
Office européen des brevets

(11) Publication number: **0 468 929 B1**

(12) **EUROPEAN PATENT SPECIFICATION**

(45) Date of publication of patent specification: **28.09.94**

(51) Int. Cl.⁵: **A61K 47/18**, A61K 47/02, A61K 9/20

(21) Application number: **91810591.7**

(22) Date of filing: **23.07.91**

(54) **Stabilized pharmaceutical compositions.**

(30) Priority: **25.07.90 US 557234**

(43) Date of publication of application:
**29.01.92 Bulletin 92/05**

(45) Publication of the grant of the patent:
**28.09.94 Bulletin 94/39**

(84) Designated Contracting States:
**BE DK ES GR NL SE**

(56) References cited:
**EP-A- 0 099 239**
**EP-A- 0 280 999**
**EP-A- 0 302 729**
**FR-A- 2 318 636**
**US-A- 4 900 559**

(73) Proprietor: **SANDOZ LTD.**
**Lichtstrasse 35**
**CH-4002 Basel (CH)**

(72) Inventor: **Ross, Bruce Allan**
**401 Ridge Road**
**Fredon, N.J. 07860 (US)**
Inventor: **Vivilecchia, Richard Victor**
**155 Andrea Drive**
**Rockaway, N.J. 07866 (US)**

**EP 0 468 929 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the use of certain acid donors as stabilizers in pharmaceutical compositions, and to the pharmaceutical compositions resulting therefrom.

### Background of the Invention

There are a number of pharmaceutical compositions which suffer from instability problems due to the fact that the active component is susceptible to certain types of degradation, thereby diminishing their attractiveness and, in some cases, rendering them unsuitable from a commercial standpoint. For example, several ACE (Angiotensin Converting Enzyme) inhibitor-containing compositions suffer from this drawback since certain ACE inhibitors degrade readily in pharmaceutical dosage forms. More particularly, and as is the case with other ACE inhibitors such as Quinapril and Enalapril, Spirapril may degrade in dosage forms to the diketo piperazine (the internal cyclization product) and the diacid (the ester hydrolysis product). Accordingly, in view of their usefulness in treating hypertension, a number of research endeavors have been directed to overcoming the instability problem associated with ACE inhibitor-containing compositions.

### Description of the prior art

European Patent Application 264,888 is directed to the stabilization of ACE inhibitor-containing pharmaceutical compositions employing ascorbic acid alone or a combination of ascorbic acid with fumaric acid, maleic acid and/or citric acid as the stabilizing component(s).

USP 4,743,450 is also directed to the stabilization of ACE inhibitor-containing pharmaceutical compositions employing, as the stabilizer component, a combination of an alkali or alkaline earth metal salt (preferably, magnesium carbonate) and a saccharide (preferably, mannitol or lactose).

Although each of the above patents represents an attempt to overcome the instability problems associated with ACE inhibitor-containing compositions, there still exists a dire need for ACE inhibitor-containing compositions exhibiting improved stability, especially in the presence of moisture. To this end, the present invention is directed to pharmaceutical compositions, particularly ACE inhibitor-containing compositions, exhibiting improved stability.

### DESCRIPTION OF THE INVENTION

Accordingly in one aspect the present invention provides

a) a pharmaceutical composition comprising an ACE inhibitor and a hydrochloric acid donor that releases hydrochloric acid, and

b) a process for the production of a stabilized pharmaceutical composition which comprises working up an ACE inhibitor with a hydrochloric acid donor that releases hydrochloric acid, and

c) use of a hydrochloric acid donor to stabilizer an ACE inhibitor and/or use of a hydrochloric acid donor as stabilizing agent in the manufacture of a pharmaceutical composition containing an ACE inhibitor , and

d) a method of stabilizing a pharmaceutical composition as defined above containing an ACE inhibitor comprising incorporating therein a stabilizing effective amount of a hydrochloric acid donor that releases hydrochloric acid.

The pharmaceutical compositions of the instant invention may exhibit advantages as follows:-

1) The ACE inhibitor may be preserved from degradation, and/or

2) They may exhibit an extended shelf-life under normal storage conditions, and/or

3) They may be less sensitive to moisture and even the stability may improve with an increase in moisture, and/or

4) They may exhibit less discoloration over a significant period of time, and/or

5) They may exhibit less instability when employed in the presence of colorants.

In comparison to certain acidifiers which have previously been employed as stabilizers in pharmaceutical compositions, e.g. citric acid, maleic acid, ascorbic acid, etc., the acid donors that are chosen are those which release the more volatile hydrochloric acid and, therefore, effect a greater diffusion through the dosage form matrix. Although any compounds which produce hydrochloric acid would be suitable in the practice of the instant invention, preferred acid donors include an amino acid hydrochloride, such as glycine hydrochloride, glutamic acid hydrochloride, betaine hydrochloride, alanine hydrochloride, valine hydrochloride, lysine hydrochloride, arginine hydrochloride, aspartic acid hydrochloride and a Lewis acid chloride, such as ferric chloride, zinc chloride and aluminium chloride.

The more preferred acid donors are glycine hydrochloride, glutamic acid hydrochloride and betaine hydrochloride. The most preferred acid donor is glycine hydrochloride.

Other preferred hydrochloric acid donors may be chosen e.g. on the basis of vapour pressure measurements determining the release of hydrochloric acid, e.g. having similar properties to the preferred acid donors mentioned above.

Although, in general, the hydrochloric acid donor may be employed in any amount which will prevent degradation of the ACE inhibitor , e.g. as indicated by standard stability tests, the amount of the hydrochloric acid donor employed is between 1% and 25%, preferably between 1% and 20%, more preferably between 1% and 15%, e.g. from 1 to 10%, or 1 to 5%, e.g. 2% based on the total weight of the pharmaceutical composition.

The weight ratio of ACE inhibitor to the hydrochloric acid donor may be determined in conventional manner. The preferred weight ratio of ACE inhibitor to the hydrochloric acid donor is 2.5:1 to 1:7, more preferably 2:1 to 1:2.

The instant invention, viz., the use of a select group of hydrochloric acid donors as stabilizers in pharmaceutical compositions, applies to all ACE inhibitors in pharmaceutical compositions where buffering to a low pH for required stability is desired. It may be of particular interest for ACE inhibitors which are in acid addition salt form, e.g. hydrochloride salt form. The invention has particularly been found useful when applied to ACE inhibitor-containing pharmaceutical compositions since, as indicated above, many ACE inhibitors degrade readily in pharmaceutical dosage forms. In general, ACE inhibitor-containing pharmaceutical compositions wherein ACE inhibitor employed is prone to form diketopiperazine degradation products would benefit from the use of a select group of hydrochloric acid donors as stabilizers therefore. For example, one class of ACE inhibitors to which the instant invention would apply are compounds of formula I:

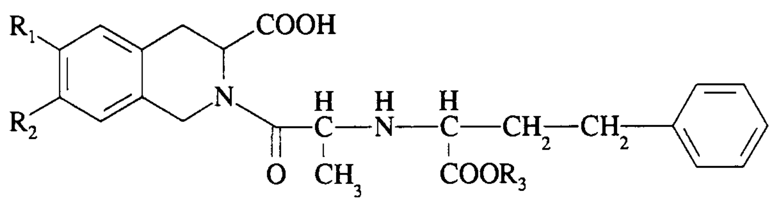

I

wherein:

$R_1$ and $R_2$, independently, are hydrogen or a group $-OC_nH_{2n+1}$, where n is 1 to 5; and

$R_3$ is hydrogen or a group $-C_nH_{2n+1}$, where n is as defined above.

In the above formula, preferred compounds are those where $R_1$ and $R_2$ have the same significance. More preferred compounds of the above formula are those where $R_1$ and $R_2$ are both hydrogen or methoxy and $R_3$ is hydrogen or methyl. The most preferred compound of the above formula is Quinapril having the formula

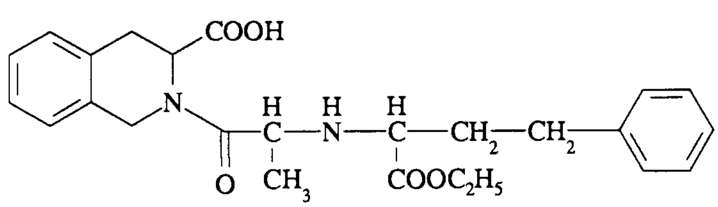

All of the above compounds are known, having been previously described e.g. in USP 4,344,949. Moreover, their usefulness in treating hypertension as well as methods for their preparation are set forth therein.

Another class of ACE inhibitors to which the invention would apply are compounds of formula II:

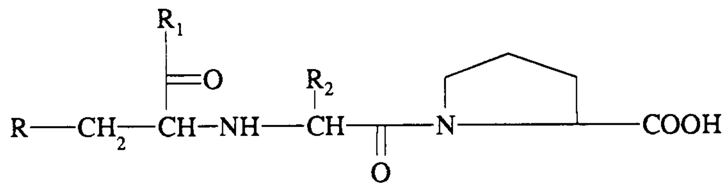

II

wherein:

R is $C_1$-$C_6$ alkyl, benzyl, benzylthio, benzyloxy, phenylthio or phenoxy;

$R_1$ is hydroxy or $C_1$-$C_6$ alkoxy; and

$R_2$ is hydrogen, $C_1$-$C_6$ alkyl or $C_1$-$C_6$ aminoalkyl.

In the above formula, preferred compounds are those wherein R is benzyl, $R_1$ is $C_1$-$C_6$ alkoxy and $R_2$ is hydrogen, methyl or aminobutyl. More preferred compounds of the above formula are those wherein R is benzyl, $R_1$ is $C_1$-$C_4$ alkoxy and $R_2$ is hydrogen or methyl. The even more preferred compounds of the above formula are those wherein R is benzyl, $R_1$ is ethoxy and $R_2$ is methyl. The most preferred compound of the above formula is Enalapril having the formula

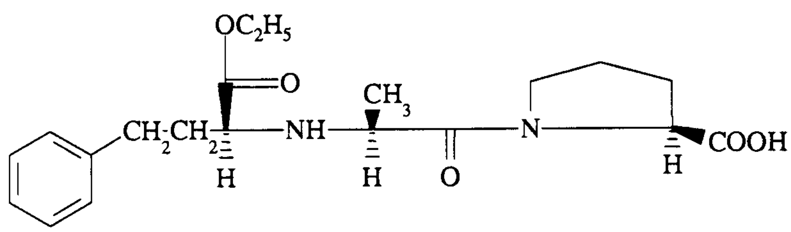

All of the above compounds of formula II are known, having been previously described e.g. in European Patent 12,401. Moreover, their usefulness in treating hypertension as well as methods for their preparation are set forth therein.

A particularly preferred class of ACE inhibitors to which the instant invention would apply are compounds of formula III:

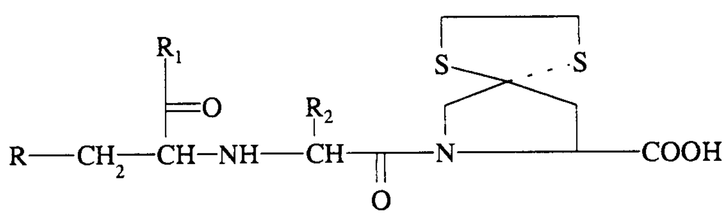

III

wherein R, $R_1$ and $R_2$ have the significances indicated above regarding the compounds of formula II.

The most preferred compound of the above formula is Spirapril having the formula

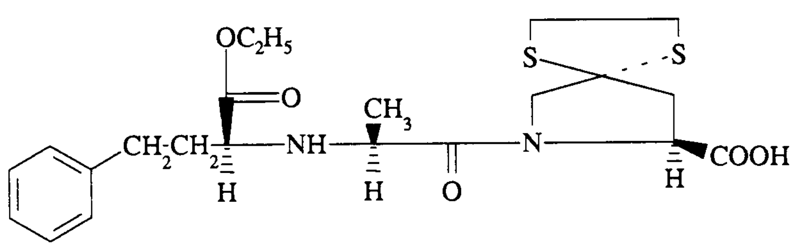

All of the above compounds of formula III are known, having been previously described e.g. in USP 4,470,972. Moreover, their usefulness in treating hypertension as well as methods for their preparation are set forth therein.

It should be noted that all of the compounds of formulae I, II and III form salts with various inorganic and organic acids and bases, which salts may be prepared by conventional methods. Therefore, it should be understood that all of such salts would also benefit from the use of the select group of hydrochloric acid donors as stabilizers therefor in accordance with the instant invention.

The amount of the ACE inhibitor in the pharmaceutical compositions of the instant invention is between 0.5% and 50%, preferably between 0.75% and 25%, e.g. between 1% and 25%, more preferably between 0.75% and 20%, e.g. between 1% and 20%, most preferably between 0.75% and 15%, e.g. between 1% and 15%, based on the total weight of the pharmaceutical composition.

As indicated above, all of the compounds of formulae I, II and III are known and their usefulness in daily dosages at which said compounds are employed as well as typical unit dosages of said compounds are well documented in the literature.

Although the pharmaceutical compositions may be in any form, the solid forms are preferred, more preferably tablets, capsules and caplets.

In addition to the ACE inhibitor, and the stabilizing component, e.g., glycine hydrochloride, the pharmaceutical compositions of the instant invention will typically contain a pharmaceutically acceptable carrier. Generally, they are compounds which do not contain groups which would significantly interfere with either the active component or the stabilizing component. For example, sugars such as lactose, sucrose, mannitol and sorbitol are quite suitable; as are starches such as corn starch and tapioca starch; cellulose and derivatives thereof such as sodium carboxymethyl cellulose, ethyl cellulose and methyl cellulose; calcium phosphates such as dicalcium phosphate; sodium sulfate; and polyvinyl alcohol. Such type compounds are generally present in amounts of between 5% and 90%, preferably between 10% and 80%, based on the total weight of the pharmaceutical composition.

The stabilized compositions of the instant invention may also contain optional ingredients that are normally employed in pharmaceutical formulations, the only qualification being that they must be compatible with the select group of hydrochloric acid donors so as not to interfere with their stabilizing function. Typical optional ingredients include lubricants, e.g., talc, alkaline earth metal stearates such as magnesium stearate and calcium stearate, and hydrogenated vegetable oils such as hydrogenated cottonseed oil; binders such as polyvinylpyrrolidone (povidone) and gelatin; and disintegrants such as microcrystalline cellulose, cross-linked polyvinylpyrrolidone and alginic acid. Other optional ingredients are fillers, water scavengers, buffers, preservatives, anti-oxidants, colorants and flavouring agents. The total amount of the optional ingredients in the stabilized compositions of the instant invention is not critical. In general, the total amount of the optional ingredients is consistent with the amount of the active component, stabilizer and pharmaceutically acceptable carrier, i.e., the total amount will be equivalent to the remainder of the pharmaceutical compositions.

The stabilized compositions of the instant invention can be prepared by any of the conventionally employed processing techniques such as the wet granulation process. The technique is preferably chosen to ensure a homogeneous distribution of the active component and a homogeneous distribution of the hydrochloric acid donor over or among the active component particles. Conveniently the hydrochloric acid donor is distributed in a liquid form, e.g. an aqueous solution used as a granulating liquid.

Examples of other active components that are contemplated include those with a -NH-CH-CO-N-C-COOH moiety as in the above formula I, II,and III, e.g the di-acid form of spirapril, spiraprilate. Such compounds include ramipril, perindopril, indolapril, lisinopril, alacepril, trandolapril, benazapril, libenzapril, delapril, and cilazapril.

The following examples are for the purpose of illustration only and are not intended in any way to limit the scope of the instant invention.

**EXAMPLE 1:**

Below are stabilized compositions in accordance with the instant invention in white tablet form:

| Ingredient | Amount (mg) | |
|---|---|---|
| | A | B |
| Quinapril hydrochloride | 40.0 | - |
| Enalapril hydrochloride | - | 40.0 |
| glycine hydrochloride | 40.0 | 40.0 |
| lactose | 277.5 | 277.5 |
| corn starch | 25.0 | 25.0 |
| talc | 15.0 | 15.0 |
| magnesium stearate | 2.5 | 2.5 |
| | 400.0 | 400.0 |

**EXAMPLE 2:**

The following compositions A-D represent stabilized compositions in accordance with the instant invention in white tablet form whereas composition E does not contain a stabilizer of the instant invention:

| Ingredient | Amount (mg) | | | | |
|---|---|---|---|---|---|
| | A | B | C | D | E |
| Spirapril hydrochloride | 3.06 | 3.06 | 3.06 | 3.06 | 3.19 |
| lactose, NF | 99.94 | 94.74 | 99.94 | 94.74 | 80.21 |
| starch,NF | 19.50 | 19.50 | 19.50 | 19.50 | 12.00 |
| povidone, USP | 2.60 | 2.60 | 2.60 | 2.60 | 2.00 |
| glycine hydrochloride | - | - | 2.60 | 2.60 | - |
| glutamic acid hydrochloride | 2.60 | 2.60 | - | - | - |
| silica gel, NF | - | 5.20 | - | 5.20 | 1.90 |
| colloidal $SiO_2$, NF | 1.30 | 1.30 | 1.30 | 1.30 | 0.10 |
| magnesium stearate, NF | 1.00 | 1.00 | 1.00 | 1.00 | 0.60 |
| total | 130.00 | 130.00 | 130.00 | 130.00 | 100.00 |

**EXAMPLE 3:**

To demonstrate the effectiveness of the stabilizers of the instant invention against added moisture, the following results were obtained when the compositions of Example 2A-2D were stored for 3 months at 30 °C and 75% relative humidity:

| | * Assay % | Diketo | Diacid |
|---|---|---|---|
| Example 2A | 99.6 | 0.0 | 0.1 |
| Example 2B | 100.0 | 0.0 | 0.2 |
| Example 2C | 99.6 | 0.0 | 0.1 |
| Example 2D | 99.9 | 0.0 | 0.2 |

* per cent of original Spirapril hydrochloride content

**EXAMPLE 4:**

To demonstrate the effectiveness of the stabilizers of the instant invention against an increase in temperature, the following results were obtained when the compositions of Example 2A and 2C were stored at 50 ° C for varying periods of time. For purposes of comparison, below are the results obtained when the composition of Example 2E was stored at 50 ° C for three months.

| | Period (months) | * Assay % | Diketo | Diacid |
|---|---|---|---|---|
| Example 2A | 1 | 99.0 | 0.2 | 0.1 |
| | 2 | 100.8 | 0.6 | 0.3 |
| | 3 | 99.1 | 0.9 | 0.3 |
| Example 2C | 1 | 100.3 | 0.1 | 0.2 |
| | 2 | 101.3 | 0.8 | 0.2 |
| | 3 | 98.4 | 1.0 | 0.3 |
| Example 2E | 3 | 91.2 | 7.3 | 0.4 |

* per cent of original Spirapril hydrochloride content

**EXAMPLE 5:**

The following compositions A and B and D represent stabilized compositions in accordance with the instant invention in colored tablet form whereas composition C contains maleic acid, an acidifier disclosed in the prior art:

| Ingredient | Amount (mg) | | | |
|---|---|---|---|---|
| | A | B | C | D |
| Spirapril hydrochloride | 3.06 | 3.06 | 3.06 | 6 |
| lactose, NF | 96.94 | 96.94 | 96.94 | 99.77 |
| starch, NF | 19.50 | 19.50 | 19.50 | 22.50 |
| povidone, USP | 2.60 | 2.60 | 2.60 | 3.0 |
| alginic acid | - | - | - | 13.0 |
| glycine hydrochloride | 2.60 | - | - | 3.0 |
| glutamic acid hydrochloride | - | 2.60 | - | - |
| maleic acid | - | - | 2.60 | - |
| carmine | 3.00 | 3.00 | 3.00 | - |
| iron oxide, red | - | - | - | 0.03 |
| colloidal $SiO_2$, NF | 1.30 | 1.30 | 1.30 | 1.5 |
| magnesium stearate, NF | 1.00 | 1.00 | 1.00 | 1.2 |
| total | 130.00 | 130.00 | 130.00 | 150.00 |

Representative production process

A batch of 1.6 million tablets of Example 5D is made as follows:

a) Spirapril hydrochloride (4.8 kg), 79.576 kg lactose, starch (18 kg) and povidone (2.4 kg) are sieved separately (typically 1600 µm) and mixed together in a high speed mixer.

b) Iron oxide (0.024 kg) and lactose (0.24 kg) are mixed together and sieved, and added to the mixture obtained in step a).

c) Glycine hydrochloride (2.4 kg) in 13.40 kg of demineralised water is pumped into the mixture obtained in step b), mixed and kneaded until suitable for granulation. The granulate is dried in a fluidized bed drier at 60 °C e.g. until a loss on drying of about 2.1% is reached, then broken by sieving (typically 1000 µm). A batch of 107.44 kg results.

d) A second batch of 107.44 kg is produced following steps a), b) and c), and the batches are combined (214.88 kg).

e) In another vessel 20.8 kg alginic acid, 2.4 kg colloidal $SiO_2$ are mixed together and sieved (typically 1000 µm) and sieved with almost the whole of the granulate obtained in step d).

f) 1.92 kg magnesium stearate and the remaining part of the granulate obtained in step d) are mixed together, sieved (typically 1000 µm) and combined with the mixture in step e). 240 kg of tabletting mixture is obtained and this is compressed into tablets.

**EXAMPLE 6:**

To demonstrate the effectiveness of the stabilizers of the instant invention against an increase in temperature in the presence of colorants, the following results were obtained when the carmine colored compositions of Example 5A and 5B were stored at 50 °C for three months.

| | * Assay % | Diketo | Diacid |
|---|---|---|---|
| Example 5A | 96.3 | 2.7 | ** |
| Example 5B | 96.0 | 1.8 | ** |

* per cent of original Spirapril hydrochloride content

** interference from dye

**EXAMPLE 7:**

To show the superiority of the pharmaceutical compositions of the present invention, the following results were obtained when the compositions of Example 5A and 5C were stored at 50°C for varying periods of time:

| | Period (months) | * Assay %) | Diketo | ** Diacid |
|---|---|---|---|---|
| Example 5A | 1 | 98.4 | 1.1 | 1 |
| | 2 | 15.2 | 3.1 | 2 |
| | 3 | 96.3 | 2.7 | 2 |
| Example 5C | 1 | 91.6 | 5.1 | 1 |
| | 2 | 89.2 | 14.8 | 2 |
| | 3 | 84.6 | 10.0 | 2 |

* per cent of original Spirapril hydrochloride content

** interference from dye

**EXAMPLE 8:**

The following compositions A-D represent stabilized compositions in accordance with the instant invention in white tablet form whereas composition E does not contain a stabilizer of the instant invention:

| Ingredient | Amount (mg) | | | | |
|---|---|---|---|---|---|
| | A | B | C | D | E |
| Spirapril hydrochloride | 3.0 | 3.3 | 3.3 | 3.3 | 3.3 |
| lactose, NF | 360.0 | 360.0 | 360.0 | 360.0 | 360.0 |
| glycine hydrochloride | 20.0 | - | - | - | - |
| ferric chloride | - | 20.0 | - | - | - |
| betaine hydrochloride | - | - | 20.0 | - | - |
| glutamic acid hydrochloride | - | - | - | 20.0 | - |
| colloidal $SiO_2$, NF | 1.0 | 1.0 | 1.0 | 1.0 | 1.0 |
| stearic acid, NF | 16.0 | 16.0 | 16.0 | 16.0 | 16.0 |
| total | 400.3 | 400.3 | 400.3 | 400.3 | 380.3 |

**EXAMPLE 9:**

To demonstrate the effectiveness of the pharmaceutical compositions of the instant invention against an increase in temperature and added moisture, the following results were obtained when the compositions of Examples 8A-8D stored for 72 hours. For purposes of comparison, below are the results obtained when the composition of Example 8E was stored for 72 hours under the same conditions.

| | Temp.(°C) | % Water | *Assay % | Diketo | Diacid |
|---|---|---|---|---|---|
| Example 8A | 0 | 0 | 94 | 0.1 | 0.10 |
| | 65 | 0 | 91 | 0.6 | 0.05 |
| | 65 | 5 | - | - | - |
| | 65 | 10 | 92 | 0.7 | 0.10 |
| Example 8B | 0 | 0 | 62 | 0.3 | 0.80 |
| | 65 | 0 | 66 | 0.4 | 0.60 |
| | 65 | 5 | 72 | 0.7 | 1.40 |
| | 65 | 10 | 66 | 1.3 | 3.10 |
| Example 8C | 0 | 0 | 94 | 0.1 | 0.40 |
| | 65 | 0 | 91 | 4.0 | 0.03 |
| | 65 | 5 | 94 | 0.9 | 0.07 |
| | 65 | 10 | 95 | 0.8 | 0.14 |
| Example 8D | 0 | 0 | 95 | 0.2 | 0.03 |
| | 65 | 0 | 91 | 3.6 | 0.03 |
| | 65 | 5 | 97 | 0.4 | 0.10 |
| | 65 | 10 | 94 | 0.4 | 0.20 |
| Example 8E | 0 | 0 | 93 | 0.1 | 0.05 |
| | 65 | 0 | 87 | 6.0 | 0.04 |
| | 65 | 5 | 79 | 9.0 | 0.20 |
| | 65 | 10 | 65 | 17.0 | 0.30 |

**EXAMPLE 10:**

To demonstrate the extended shelf-life of a stabilized composition in accordance with the instant invention, the following results were obtained when the composition of Example 5A was stored for an extended period under various conditions:

| Period (months) | 30°C | | 40°C | | 50°C | | 30°C/75% RH | |
|---|---|---|---|---|---|---|---|---|
| | DK | DA | DK | DA | DK | DA | DK | DA |
| 0 | 0.4 | 0.0 | - | - | - | - | - | - |
| 3 | 0.4 | 0.1 | 1.3 | 0.2 | 2.5 | 0.2 | 0.4 | 0.1 |
| 6 | 0.5 | 0.2 | 1.7 | 0.2 | 3.0 | 0.1 | 0.5 | 0.1 |
| 9 | 0.9 | 0.2 | - | - | - | - | - | - |
| 12 | 1.1 | 0.2 | 2.6 | 0.1 | - | - | - | - |
| 24 | 1.5 | 0.2 | - | - | - | - | - | - |

DK = diketopiperazine

DA = diacid

**Claims**

**Claims for the following Contracting States : BE, DK, NL, SE**

1. A pharmaceutical composition comprising an ACE inhibitor and a hydrochloric acid donor that releases hydrochloric acid.

2. A composition according to claim 1 wherein the ACE inhibitor is spirapril, quinapril or enalapril.

3. A composition according to any preceding claim wherein the ACE inhibitor is in the form of a hydrochloride acid addition salt.

4. A composition according to any preceding claim wherein the hydrochloric acid donor is an amino acid hydrochloride or a Lewis acid chloride.

5. A composition according to claim 4 wherein the hydrochloric acid donor is glycine hydrochloride, glutamic acid hydrochloride, betaine hydrochloride, alanine hydrochloride, valine hydrochloride, lysine hydrochloride, arginine hydrochloride, ferric chloride, zinc chloride or aluminium chloride.

6. A composition according to claim 5 wherein the hydrochloric acid donor is glycine hydrochloride.

7. A composition according to any preceding claim wherein the hydrochloric acid donor is present in an amount between 1% and 25%, based on the total weight of the composition.

8. A composition according to any preceding claim wherein the weight ratio of ACE inhibitor to hydrochloric acid donor is from about 2.5:1 to about 1:7.

9. A composition according to any preceding claim in the form of a tablet.

10. A process for the production of a stabilized pharmaceutical composition as defined in any one of claims 1 to 9 which comprises working up an ACE inhibitor with a hydrochloric acid donor.

11. Use of a hydrochloric acid donor to stabilize an ACE inhibitor in a pharmaceutical composition as defined in any one of claims 1 to 9.

12. Use of a hydrochloric acid donor as stabilizing agent in the manufacture of a pharmaceutical composition containing an ACE inhibitor as defined in any one of claims 1 to 9.

**Claims for the following Contracting States : ES, GR**

1. A process for the production of a stable pharmaceutical composition comprising an ACE inhibitor, the process comprising mixing the ACE inhibitor and a hydrochloric acid donor that releases hydrochloric acid, and formulating into a pharmaceutical composition.

2. A process according to claim 1 wherein the ACE inhibitor is spirapril, quinapril or enalapril.

3. A process according to any preceding claim wherein the ACE inhibitor is in the form of a hydrochloride acid addition salt.

4. A process according to any preceding claim wherein the hydrochloric acid donor is an amino acid hydrochloride or a Lewis acid chloride.

5. A process according to claim 4 wherein the hydrochloric acid donor is glycine hydrochloride, glutamic acid hydrochloride, betaine hydrochloride, alanine hydrochloride, valine hydrochloride, lysine hydrochloride, arginine hydrochloride, ferric chloride, zinc chloride or aluminium chloride.

6. A process according to claim 5 wherein the hydrochloric acid donor is glycine hydrochloride.

7. A process according to any preceding claim wherein the hydrochloric acid donor is present in an amount between 1% and 25%, based on the total weight of the composition.

8. A process according to any preceding claim wherein the weight ratio of ACE inhibitor to hydrochloric acid donor is from about 2.5:1 to about 1:7.

9. A process according to any preceding claim in which the pharmaceutical composition is formulated into a tablet.

10. Use of a hydrochloric acid donor to stabilize an ACE inhibitor in a pharmaceutical composition as defined in any one of claims 1 to 9.

11. Use of a hydrochloric acid donor as stabilizing agent in the manufacture of a pharmaceutical composition containing an ACE inhibitor as defined in any one of claims 1 to 9.

**Patentansprüche**
**Patentansprüche für folgende Vertragsstaaten : BE, DK, NL, SE**

1. Eine pharmazeutische Zusammensetzung enthaltend einen ACE Hemmer und einen Chlorwasserstoffsäure Donator, der Chlorwasserstoffsäure freisetzt.

2. Eine Zusammensetzung gemäss Anspruch 1, worin der ACE Hemmer Spirapirl, Chinapril oder Enalapril ist.

3. Eine Zusammensetzung gemäss einem der vorhergehenden Ansprüche, worin sich der ACE Hemmer in Form eines Chlorwasserstoffsäure Additionssalzes befindet.

4. Eine Zusammensetzung gemäss einem der vorhergehenden Ansprüche, worin der Chlorwasserstoffsäure Donator ein Aminosäure hydrochlorid oder ein Lewissäurechlorid ist.

5. Eine Zusammensetzung gemäss Anspruch 4, worin der Chlorwasserstoffsäure Donator, Glycin hydrochlorid, Glutaminsäure hydrochlorid, Betain hydrochlorid, Alanin hydrochlorid, Valin hydrochlorid, Lysin hydrochlorid, Arginin hydrochlorid, Eisen(III)chlorid, Zinkchlorid oder Aluminiumchlorid ist.

6. Eine Zusammensetzung gemäss Anspruch 5, worin der Chlorwasserstoffsäure Donator, Glycin hydrochlorid ist.

7. Eine Zusammensetzung gemäss einem der vorhergehenden Ansprüche, worin der Chlorwasserstoffsäure Donator in einem Antiel zwischen 1% und 25%, bezogen auf das Gesamtgewicht der Zusammensetzung, anwesend ist.

8. Eine Zusammensetzung gemäss einem der vorhergehenden Ansprüche, worin das Gewichtsverhältnis des ACE Hemmers zum Chlorwasserstoffsäure Donator von ungefähr 2,5:1 bis zu ungefährt 1:7 beträgt.

9. Eine Zusammensetzung gemäss einem der vorhergehenden Ansprüche in Form einer Tablette.

10. Ein Verfahren zur Herstellung einer stabilisierten pharmazeutischen Zusammensetzung, wie in einem der Ansprüche 1 bis 9 definiert, das die Aufarbeitung eines ACE Hemmers mit einem Chlorwasserstoffsäure Donator umfasst.

11. Verwendung eines Chlorwasserstoffsäure Donators zur Stabilisierung eines ACE Hemmers in einer pharmazeutischen Zusammensetzung, wie in den Ansprüchen 1 bis 9 definiert.

12. Verwendung eines Chlorwasserstoffsäure Donators als Stabilisierungsmittel bei der Herstellung einer pharmazeutischen Zusammensetzung enthaltend einen ACE Hemmer, wie in einem der Ansprüche 1 bis 9 definiert.

**Patentansprüche für folgende Vertragsstaaten : ES, GR**

1. Ein Verfahren zur Herstellung einer stabilen pharmazeutischen Zusammensetzung enthaltend einen ACE Hemmer, das Verfahren umfassend das Vermischen des ACE Hemmers und eines Chlorwasserstoffsäure Donators der Chlorwasserstoffsäure freisetzt und Formulierung in eine pharmazeutische Zusammensetzung.

2. Ein Verfahren gemäss Anspruch 1, worin der ACE Hemmer Spirapril, Chinapril oder Enalapril ist.

3. Ein Verfahren gemäss einem der vorhergehenden Ansprüche, worin sich der ACE Hemmer in Form eines Chlorwasserstoffsäure Additionssalzes befindet.

4. Ein Verfahren gemäss einem der vorhergehenden Ansprüche, worin der Chlorwasserstoffsäure Donator ein Aminosäure.hydrochlorid oder ein Lewissäure.chlorid ist.

5. Ein Verfahren gemäss Anspruch 4, worin der Chlorwasserstoffsäure Donator Glycin.hydrochlorid, Glutaminsäure.hydrochlorid, Betain.hydrochlorid, Alanin.hydrochlorid, Valin.hydrochlorid, Lysin.hydrochlorid, Arginin.hydrochlorid, Eisen(III)chlorid, Zinkchlorid oder Aluminiumchlorid ist.

6. Ein Verfahren gemäss Anspruch 5, worin der Chlorwasserstoffsäure Donator Glycin.hydrochlorid ist.

7. Ein Verfahren gemäss einem der vorhergehenden Ansprüche, worin der Chlorwasserstoffsäure Donator in Anteilen zwischen 1% und 25%, basierend auf dem Gesamtgewicht der Zusammensetzung, anwesend ist.

8. Ein Verfahren gemäss einem der vorhergehenden Ansprüche, worin das Gewichtsverhältnis des ACE Hemmers zum Chlorwasserstoffsäure Donator von ungefähr 2.5:1 bis ungefähr 1:7 beträgt.

9. Ein Verfahren gemäss einem der vorhergehenden Ansprüche, worin die pharmazeutische Zusammensetzung in eine Tablette formuliert ist.

10. Verwendung eines Chlorwasserstoffsäure Donators zur Stabilisierung eines ACE Hemmers in einer pharmazeutischen Zusammensetzung, wie in einem der Ansprüche 1 bis 9 definiert.

11. Verwendung eines Chlorwasserstoffsäure Donators als Stabilisierungsmittel bei der Herstellung einer pharmazeutischen Zusammensetzung, enthaltend einen ACE Hemmer, wie in einem der Ansprüche 1 bis 9 definiert.

**Revendications**

**Revendications pour les Etats contractants suivants : BE, DK, NL, SE**

1. Une composition pharmaceutique caractérisée en ce qu'elle comprend un inhibiteur de l'enzyme de conversion de l'angiotensine (ECA) et un donneur d' acide chlorhydrique qui libère de l'acide chlorhydrique.

2. Une composition selon la revendication 1, caractérisée en ce que l'inhibiteur de l'ECA est le spirapril, le quinapril ou l'enalapril.

3. Une composition selon l'une quelconque des revendications 1 ou 2, caractérisée en ce que l'inhibiteur de l'ECA est sous forme d'un sel d'addition de l'acide chlorhydrique.

4. Une composition selon l'une quelconque des revendications 1 à 3, caractérisée en ce que le donneur d'acide chlorhydrique est un chlorhydrate d'acide aminé ou un chlorure d'acide de Lewis.

5. Une composition selon la revendication 4, caractérisée en ce que le donneur d' acide chlorhydrique est le chlorhydrate de glycine, le chlorhydrate de l'acide glutamique, le chlorhydrate de bétàïne, le chlorhydrate d'alanine, le chlorhydrate de valine, le chlorhydrate de lysine, le chlorhydrate d' arginine, le chlorure ferrique, le chlorhre de zinc ou le chlorure d'aluminium.

6. Une composition selon la revendication 5, caractérisée en ce que le donneur d'acide chlorhydrique est le chlorhydrate de glycine.

7. Une composition selon l'une quelconque des revendications 1 à 6, caractérisée en ce que le donneur d'acide chlorhydrique est présent en une proportion comprise entre 1% et 25% par rapport au poids total de la composition.

8. Une composition selon l'une quelconque des revendications 1 à 7, caractérisée en ce que le rapport pondéral de l'inhibiteur l'ECA au donneur d'acide chlorhydrique est d'environ 2,5:1 à environ 1:7.

9. Une composition selon l'une quelconque des revendications 1 à 8, caractérisée en ce qu'elle se présente sous forme de comprimés.

10. Un procédé de préparation d'une composition pharmaceutique stabilisée telle que définie à l'une quelconque des revendications 1 à 9, caractérisé en ce qu'il comprend le traitement d'un inhibiteur l'ECA par un donneur d'acide chlorhydrique.

11. Utilisation d'un donneur d'acide chlorhydrique pour stabiliser un inhibiteur de l'ECA dans une composition pharmaceutique telle que définie à l'une quelconque des revendications 1 à 9.

12. Utilisation d'un donneur d'acide chlorhydrique comme agent de stabilisation pour la préparation d'une composition pharmaceutique contenant un inhibiteur de l'ECA tel que défini à l'une quelconque des revendications 1 à 9.

**Revendications pour les Etats contractants suivants : ES, GR**

1. Un procédé de préparation d'une composition pharmaceutique stable comprenant un inhibiteur de l'enzyme de conversion de l'angiotensine (ECA), caractérisé en ce qu'on mélange l'inhibiteur de l'ECA et un donneur d'acide chlorhydrique qui libère de l'acide chlorhydrique et on les formule en une composition pharmaceutique.

2. Un procédé selon la revendication 1, caractérisé en ce que l'inhibiteur de l'ECA est le spirapril, le quinapril ou l'enalapril.

3. Un procédé selon la revendication 1 ou 2, caractérisé en ce que l'inhibiteur de l'ECA est sous forme d'un sel d'addition de l' acide chlorhydrique.

4. Un procédé selon l'une quelconque des revendications 1 à 3, caractérisé en ce que le donneur d' acide chlorhydrique est un chlorhydrate d'acide aminé ou un chlorure d' acide de Lewis.

5. Un procédé selon la revendication 4, caractérisé en ce que le donneur d'acide chlorhydrique est le chlorhydrate de glycine, le chlorhydrate de l'acide glutamique, le chlorhydrate de bétaïne, le chlorhydrate d'alanine, le chlorhydrate de valine, le chlorhydrate de lysine, le chlorhydrate d'arginine, le chlorure ferrique, le chlorhre de zinc ou le chlorure d' aluminium.

6. Un procédé selon la revendication 5, caractérisé en ce que le donneur d'acide chlorhydrique est le chlorhydrate de glycine.

7. Un procédé selon l'une quelconque des revendications 1 à 6, caractérisé en ce que le donneur d'acide chlorhydrique est présent en une proportion comprise entre 1% et 25% par rapport au poids total de la composition.

8. Un procédé selon l'une quelconque des revendications 1 à 7, caractérisé en ce que le rapport pondéral de l'inhibiteur de l'ECA au donneur d'acide chlorhydrique est d'environ 2,5:1 à environ 1:7.

9. Un procédé selon l'une quelconque des revendications 1 à 8, caractérisé en ce que la composition pharmaceutique se présente sous forme de comprimés.

10. Utilisation d'un donneur d'acide chlorhydrique pour stabiliser un inhibiteur de l'ECA dans une composition pharmaceutique telle que définie à l'une quelconque des revendications 1 à 9.

11. Utilisation d'un donneur d'acide chlorhydrique comme agent de stabilisation pour la préparation d'une composition pharmaceutique contenant un inhibiteur de l'ECA tel que défini à l'une quelconque des revendications 1 à 9.